# EUROPEAN PATENT SPECIFICATION

(11) **EP 1 338 273 B1**
(45) Date of publication and mention of the grant of the patent: **07.06.2017**
(21) Application number: 02079497.0
(22) Date of filing: 01.12.1999
(51) Int. Cl.: A61K 9/14

(54) **Crystalline triamcinolone acetonide produced by a milling process**
Durch einen Mahlprozess produziertes, kristallines Triamcinolon acetonid
L'acétonide de triamcinolone cristalline produit par un procédé de broyage

(30) Priority: 01.12.1998 GB 9826286
(43) Date of publication of application: 27.08.2003
(62) Divisional of application: 99972947.8
(73) Proprietor: Aventis Pharma Limited, West Malling, Kent ME19 4AH (GB)
(72) Inventor: Authelin, Jean-Rene, c/o Rhone-Poulenc Rorer SA, 94403 Cedex Vitry-sur-Seine (FR); Hosek, Patrick, Rhone-Poulenc Rorer SA, 94403 Cedex Viltry-Sur-Seine (FR)
(74) Representative: Adamson Jones

(56) References cited:
- EP-A- 0 336 787
- WO-A1-95/05805
- WO-A1-98/31352
- "The Merck Index" THE MERCK INDEX, vol. 11, 1989, XP002245590
- Rudolf Voigt: In: Rudolf Voigt: "Pharmazeutische Technologie für Studium und Beruf", 1992, ullstein pages 427-427,

## Description

The present invention relates to crystalline triamcinolone acetonide intended for use as an inhalation medicament.

Inhalation medicaments must have a fine particle size in order to penetrate deep into the lungs where they can be absorbed. Typically particles less than 10 microns in size are required. Such fine particles are normally prepared by milling the material to be inhaled. It is well known that the intensive milling required to produce these fine particle sizes can produce profound changes in the crystal structure of the material being milled. The exact changes are governed by the nature of the starting material but commonly freshly milled powders have a greatly increased content of amorphous phase. This initially forms on the surface of the particles but can constitute a large proportion of the total weight of the powder.

Changes in crystal structure, including increase in amorphous content, can cause a number of problems. The particles tend to stick together, making the freshly milled powder extremely cohesive. With time, often under the influence of ambient moisture, the surface phase tends to revert to its more stable original phase. This can cause the particles to be welded together. Furthermore, the crystal form of a pharmaceutical substance can have a significant effect on its potency, as discussed by J.I. Wells in Pharmaceutical Preformulation: The Physiochemical Properties of Drug Substances, John Wiley & Sons, New York (1988). We have now found that by careful control of the milling conditions used we can achieve the required particle size for an inhalation medicament without generating amorphous phases on the surface of the powder.

US5562923 describes a method for producing finely milled highly crystalline medicinal substances intended for use as inhalation medicaments by drying the milled medicament, treating with a non aqueous solvent and then drying. US 5637620 uses a different method; the milled medicament is conditioned under controlled conditions of temperature and humidity before being dried. WO 98/31352 relates to a dry powder composition useful in the treatment of respiratory disorders, particularly asthma, comprising one or more potent pharmaceutically active substances, including glucocorticosteroids, and a carrier substance, all of which are in finely divided form, wherein the formulation has a poured bulk density of from 0.28 to 0.38 g/ml and a preferred median particle diameter of 1-7 microns.

In a fluid energy mill the material to be milled is entrained in an airstream and the particles caused to collide with one another by turbulence in the air stream. However, the energy input to the powder surface tends to produce a phase change to an amorphous state. One possible solution to this problem would be to mill at a reduced temperature. The material to be milled is likely to be more brittle and friable, resulting in a lower energy input to each powder particle. Also phase change reactions tend to proceed more slowly at lower temperatures. To be effective temperatures well below 0⁰C are required. One problem with this approach is that the milling fluids most commonly used, nitrogen and air, become less effective as their temperature drops. In particular the exit velocity of the gas from the milling nozzles becomes too low.

We have now found that this problem can be overcome by using helium as milling fluid. The process provides finely milled, highly crystalline material containing substantially no amorphous material. A surprising advantage is that build up of scale in the mill during milling is much reduced. Less scale is deposited and the scale which is deposited is less hard and easier to remove.

Therefore, according to the present invention there is provided crystalline triamcinolone acetonide as defined in Claim 1.

Pure helium can be used or a mixture of helium and another gas. Thus, for example, nitrogen and/or air can be mixed with helium. Pure helium is preferred. Preferably the milling temperature falls within the range of -30 to -120⁰C, more preferably in the range -50 to -70⁰C.

The particle size of the product is controlled in the conventional manner by adjusting pressure and flow rate of the milling fluid and feed rate of the material to be milled. Any equipment conventionally used in combination with a fluid energy mill to help control product particle size distribution can also be used in conjunction with the claimed method. The reduced tendency to form scale is particularly advantageous when a classifier is used in conjunction with the mill.

We have also found that it is possible to produce extra fine powder by the method described above. Milled powders with a median particle size as low as 1 micron can be produced. The lower limit of powder median particle size which is produced by conventional fluid energy milling is around 2 to 3 micron.

The amount of amorphous material in a sample of milled powder can be assessed in a number of ways. Differential Scanning Calorimetry (DSC) will show the heat of crystallisation in a sample containing amorphous material. Alternatively the change in weight of a sample exposed to an atmosphere of controlled temperature and humidity can give a measure of the change in amorphous content. In both methods the apparatus is calibrated using samples of known crystalline content and the unknown sample measured by comparing the magnitude of the measurement for the unknown with the known samples.

Also, amorphous substances usually have a substantially higher specific surface area than the corresponding crystalline substance. Thus, when an powder with an appreciable amorphous content crystallises the specific surface area falls. When a powder produced by conventional milling with a substantial amorphous content is stored in contact with the atmosphere the amorphous material tends to crystallise over a period of time. Within a few days, or weeks at most, surface area falls to a substantially stable value.

Accordingly, in the context of the present invention a powder may be considered to have substantially no amorphous content if its specific surface area does not change substantially when stored in a container open to the atmosphere for a week or more. The change in surface area should preferably be no more than 20% of the initial value, more preferably no more than 10% and most preferably no more than 5%. Triamcinolone acetonide according to Claim 1 has a level of amorphous content immediately after milling as measured by weight change under controlled relative humidity or DSC of less than 1%.

Surface area can be measured by gas absorption using the Brunauer-Emmet-Teller method or by air permeametry using the Blaine method. Results given here relate to the latter method which is described in the standard method of the l'Association Francaise de Normalisation (AFNOR) no P 15-442 March 1987.

Weight change under controlled relative humidity is measured using a DVS 1 dynamic vapour sorption apparatus. A small weighed sample is placed in a microbalance pan and held at constant temperature of 25⁰C and a relative humidity of 75%. Weight change is measured as a function of time over a period of at least 5 hours. The plot of weight v time shows a peak which is proportional to the proportion of amorphous material present. The equipment is calibrated with samples of known amorphous content produced by mixing fully crystalline and fully amorphous materials.

DSC measurements were carried out using a Seiko RDC 220 system. The sample is weighed into the measuring pan and held at a temperature below the recrystallisation temperature for 30 minutes under a flow of dry nitrogen to remove any surface moisture. The sample was then heated at a constant rate of 20⁰C per minute. The exothermic peak due to recrystalisation is measured. As above the method is calibrated using samples of known amorphous content.

### Example

A two inch diameter pancake mill was used for the experiments. Helium is introduced to the circumference of the mill and powder to be milled is blown in through a venturi orifice also entering through the circumference of the milling chamber. Milled product, entrained in the milling fluid, exits through a central outlet. The temperature of the milling gas and/or the feed gas can be controlled.

The table below gives results obtained when milling triamcinolone acetonide (TAA) according to the present invention. The same feed was used in all cases and the starting material had a median particle size (d50) as measured by Malvern particle size analyser of around 25 micron. The gas used was helium or nitrogen in all cases.

Surface area was measured using the Blaine air permeability method. Where samples were stored for ageing trials the samples were kept in a 60% relative humidity atmosphere at 25⁰C. Run 1 and Run 2 (Comparative Examples) compare the effects of room temperature helium and nitrogen as milling gas. Helium gives a finer, higher surface area product but both products have a relatively high amorphous content.

Run 3 (Comparative Example) used nitrogen at -7⁰C as milling gas. Again a relatively high amorphous content was produced.

Run 4 and Run 5 (according to the invention) used cold helium as the milling and carrier gas. The product had no detectable amorphous content and was also significantly finer than would be expected given the milling conditions

| | Run 1 | Run 2 | Run 3 | Run 4 | Run 5 |
|---|---|---|---|---|---|
| Feeding rate (kg/h) | 0.1 | 1 | 0.1 | 1 | 1 |
| Milling pressure (bar) | 4 | 5 | 7 | 5 | 5 |
| Feed gas pressure (bar) | 5 | 7 | 9 | 7 | 7 |
| Gas | Helium | Nitrogen | Nitrogen | Helium | Helium |
| Temperature (°C) | Room T | Room T | - 7 | -65 | -50 |
| mill size (inches) | 2 | 4 | 2 | 4 | 4 |
| | | | | | |
| Product Sw (m²/g) | 3.2 | 1.5 | 1.2 | 3.0 | 3.3 |
| Product Sw (m²/g) after one week | - | - | - | 2.9 | - |
| Product Sw (m²/g) after two weeks | - | - | - | - | 3.3 |
| | | | | | |
| Product d50 (µm) | - | - | - | 1.5 | 1.5 |
| | | | | | |
| Amorphous content (%) | 7.6 | 3.2 | 5.8 | n.d. | n.d. |

| | | | | | |
|---|---|---|---|---|---|
| n.d. = not detected | | | | | |

Product from Run 5 was tested in an Ultrahaler® device and the results compared with product milled in the conventional way. The Ultrahaler® is a dry powder inhaler whose basic operation is described in EP 407 028.

A compact was produced by compressing a mixture of 5% milled product with 95% lactose with a median particle size of 50 micrometer. The compact is loaded into the inhaler and doses cut off from it using a blade. Up to 200 doses can be obtained from each device. The important parameters are dose uniformity and the percentage respirable fraction of medicament produced in each dose.

For product produced by conventional means the mean respirable fraction produced was 44% and 83% of the doses cut were within 20% of their nominal weight. For product produced under the conditions of Run 5 the mean respirable fraction was 40% but the percentage of doses within 20% of nominal weight rose to 90%.

## Claims

1. Crystalline triamcinolone acetonide with an amorphous content of less than 1% having a median particle size of between 1 and 2 microns.

2. Crystalline triamcinolone acetonide as claimed in claim 1 when produced by fluid energy milling of crystalline triamcinolone acetonide using a milling fluid comprising helium at a temperature within the range of -30 to -120°C.

## Patentansprüche

1. Kristallines Triamcinolonacetonid mit einem amorphen Gehalt von weniger als 1%, aufweisend eine mittlere Korngröße zwischen 1 und 2 Mikrometern.

2. Kristallines Triamcinolonacetonid nach Anspruch 1, bei Herstellung durch Mahlen von kristallinem Triamcinolonacetonid in einer Strahlmühle unter Verwendung einer Helium umfassenden Mahlflüssigkeit bei einer Temperatur im Bereich von -30 bis -120°C.

## Revendications

1. Acétonide de triamcinolone cristallin présentant une teneur en matière amorphe inférieure à 1 %, ayant une valeur de taille de particule médiane comprise entre 1 et 2 micromètres.

2. Acétonide de triamcinolone cristallin selon la revendication 1 lorsqu'il est produit par broyage par jet fluide d'acétonide de triamcinolone cristallin au moyen d'un fluide de broyage comprenant de l'hélium à une température se situant dans la plage de -30 à -120 °C.
